# EUROPEAN PATENT APPLICATION

(11) **EP 2 123 677 A1**
(43) Date of publication of application: **25.11.2009**
(21) Application number: 08711427.8
(22) Date of filing: 12.02.2008
(51) Int. Cl.: C07K 16/18, A61K 39/395, A61K 49/00, A61K 51/00, A61P 3/04, A61P 3/10, C12N 5/10, C12P 21/08, G01N 33/53

(54) **ANTI-BRAK (CXCL14) HUMAN MONOCLONAL ANTIBODY AND USE THEREOF**

(30) Priority: 09.02.2007 JP 2007030027
(71) Applicant: Tokyo Metropolitan Organization for Medical Research, Tokyo 163-8001 (JP)
(72) Inventor: HARA, Takahiko, Matsudo-shi Chiba 270-2231 (JP); TERASAWA, Yuki, Kumamoto-shi Kumamoto 862-0949 (JP)
(74) Representative: Woods, Geoffrey Corlett
(86) International application number: PCT/JP2008/052603
(87) International publication number: WO 2008/096905

(57) **Abstract**

The present invention provides an antibody that recognizes BRAK (CXCL14) and use thereof. The antibody of the invention recognizes a polypeptide comprising an amino acid sequence represented by SEQ ID NO: 1 or 2, a derivative thereof or a partial peptide thereof.

SEQUENCE LISTING

<110>  Tokyo Metropolitan Organization for Medical Research

<120>  anti-BRAK human monoclonal antibody

<130>  PCT08-0008

<150>  JP 2007-030027
<151>  2007-02-09

<160>  6

<170>  PatentIn version 3.3

<210>  1
<211>  99
<212>  PRT
<213>  Homo sapiens

<400>  1

Met Arg Leu Leu Ala Ala Ala Leu Leu Leu Leu Leu Leu Ala Leu Tyr
1               5                   10                  15

Thr Ala Arg Val Asp Gly Ser Lys Cys Lys Cys Ser Arg Lys Gly Pro
            20                  25                  30

Lys Ile Arg Tyr Ser Asp Val Lys Lys Leu Glu Met Lys Pro Lys Tyr
        35                  40                  45

Pro His Cys Glu Glu Lys Met Val Ile Ile Thr Thr Lys Ser Val Ser
    50                  55                  60

Arg Tyr Arg Gly Gln Glu His Cys Leu His Pro Lys Leu Gln Ser Thr
65                  70                  75                  80

Lys Arg Phe Ile Lys Trp Tyr Asn Ala Trp Asn Glu Lys Arg Arg Val
                85                  90                  95

Tyr Glu Glu

<210> 2
<211> 77
<212> PRT
<213> Homo sapiens

<400> 2

Ser Lys Cys Lys Cys Ser Arg Lys Gly Pro Lys Ile Arg Tyr Ser Asp
1               5                   10                  15

Val Lys Lys Leu Glu Met Lys Pro Lys Tyr Pro His Cys Glu Glu Lys
            20                  25                  30

Met Val Ile Ile Thr Thr Lys Ser Val Ser Arg Tyr Arg Gly Gln Glu
        35                  40                  45

His Cys Leu His Pro Lys Leu Gln Ser Thr Lys Arg Phe Ile Lys Trp
    50                  55                  60

Tyr Asn Ala Trp Asn Glu Lys Arg Arg Val Tyr Glu Glu
65                  70                  75

<210> 3
<211> 12
<212> PRT
<213> Homo sapiens

<400> 3

Lys Cys Lys Cys Ser Arg Lys Gly Pro Lys Ile Arg
1               5                   10

<210> 4
<211> 99
<212> PRT
<213> Mus musculus

<400> 4

Met Arg Leu Leu Ala Ala Ala Leu Leu Leu Leu Leu Leu Ala Leu Cys
1               5                   10                  15

Ala Ser Arg Val Asp Gly Ser Lys Cys Lys Cys Ser Arg Lys Gly Pro
            20                  25                  30

Lys Ile Arg Tyr Ser Asp Val Lys Lys Leu Glu Met Lys Pro Lys Tyr
        35                  40                  45

Pro His Cys Glu Glu Lys Met Val Ile Val Thr Thr Lys Ser Met Ser
    50                  55                  60

Arg Tyr Arg Gly Gln Glu His Cys Leu His Pro Lys Leu Gln Ser Thr
65                  70                  75                  80

Lys Arg Phe Ile Lys Trp Tyr Asn Ala Trp Asn Glu Lys Arg Arg Val
                85                  90                  95

Tyr Glu Glu

<210> 5
<211> 77
<212> PRT
<213> Mus musculus

<400> 5

Ser Lys Cys Lys Cys Ser Arg Lys Gly Pro Lys Ile Arg Tyr Ser Asp
1               5                   10                  15

Val Lys Lys Leu Glu Met Lys Pro Lys Tyr Pro His Cys Glu Glu Lys
            20                  25                  30

Met Val Ile Val Thr Thr Lys Ser Met Ser Arg Tyr Arg Gly Gln Glu
        35                  40                  45

His Cys Leu His Pro Lys Leu Gln Ser Thr Lys Arg Phe Ile Lys Trp
    50                  55                  60

Tyr Asn Ala Trp Asn Glu Lys Arg Arg Val Tyr Glu Glu
65                  70                  75

<210> 6
<211> 12
<212> PRT
<213> Mus musculus

<400> 6

Lys Cys Lys Cys Ser Arg Lys Gly Pro Lys Ile Arg
1               5                   10

## Description

### FIELD OF THE INVENTION

The present invention relates to an antibody that recognizes BRAK (Breast and Kidney-expressed chemokine) and to use thereof.

### BACKGROUND OF THE INVENTION

Recently, the number of diabetes patients whose onset is closely related to obesity (particularly, "type 2 diabetes" that shows insulin resistance) has been increasing worldwide. Accompanying this increase is the prevailing trends in hypertension, arteriosclerosis and the like. The pathogenic mechanisms for them presumably result from general disturbances in the fat metabolism and hormonal regulation, which are now collectively referred to as "metabolic syndrome".

According to recent reports, gene expression analyses using white adipose tissue from obese mice and obese patients found that macrophages in white adipose tissue play a key role in obesity and the accompanying development of insulin resistance (see non-patent documents 1 and 2). In experiments with obese mice, first, TNFα was produced from their macrophages and suppressed glucose absorption by insulin. At the same time, secretions of inflammatory cytokines such as IL-1 and IL-6 increased, leading to chronic inflammation (see non-patent documents 3 and 4). In fact, in mice deficient in TNFα or a receptor thereof, insulin resistance caused by obesity was ameliorated (see non-patent document 5).

Recent studies suggest that activations of kinase JNK and ER stress signal transduction pathways are essential for insulin resistance caused by obesity (see non-patent documents 6 and 7). In addition, blockage of IKK-β/NF-κB pathway in the liver has also been reported to ameliorate the insulin resistance caused by obesity (see non-patent documents 8 and 9). Hence, activation of the ER stress and the JNK pathway or the IKK-β/NF-κB pathway mentioned above caused by increased secretion of inflammatory cytokine from obese adipose tissue or load of fatty acid due to macrophage infiltration is assumed to be the major cause of the development of insulin resistance associated with obesity.

Furthermore, it is also reported that production of adiponectin, a crucial secretory hormone for regulating lipid and glucose energy metabolism, is suppressed in adipose tissue of obesity mice (see non-patent document 10).

"BRAK" (Breast and Kidney-expressed chemokine) was cloned by Hromas et al. in 1999 as a novel human chemokine gene whose expression is abrogated in tumor cell lines (see non-patent document 11). Later, several groups reported analysis results for the same chemokine molecule (see non-patent documents 12-14).

So far, more than 50 types of chemokine genes have been found encoded on a mammalian chromosome. These chemokines consist of 92-99 amino acids (with a molecular weight of about 8,000-10,000), each containing four cysteine residues that form disulfide bonds. The cysteine residues and the amino acid sequence nearby the residues at the N-termini are the characteristic features of the chemokines, based on which chemokines are classified into four groups, namely "CC", "CXC", "CX₃C" and "C" (see non-patent document 15). Sixteen types of CXC chemokines, in particular, have been identified so far and classified into ELR⁺ CXC and ELR⁻ CXC chemokines according to the presence or the absence of Glu-Leu-Arg sequence (ELR sequence) at the N-termini. BRAK belongs to ELR⁻ CXC chemokines without the ELR sequence and is universally referred to as "CXCL14".

Recent studies revealed that BRAK has an activity of allowing specific migration of human peripheral blood monocyte-derived macrophages activated by prostaglandin E₂ (see non-patent document 16). BRAK was also reported to allow specific migration of dendritic precursor cells whose differentiation was induced by human monocytes and hematopoietic precursor cells (see non-patent documents 17 and 18) and human breast cancer-derived epithelial cell lines (see non-patent document 19). So BRAK is a unique chemokine that induces migration of tissue macrophages, dendritic precursor cells or the like rather than T cells, B cells or the like (see non-patent document 16) and no other CXC chemokine is known to have the same action spectrum.

Expression of BRAK has been confirmed in the small intestine, kidney, liver, uterus, mammary gland and the like of human (see non-patent document 14). Interestingly, BRAK expression was abrogated in uterus or breast cancer patients' specimens and cancer cell lines (see non-patent documents 11 and 12). This finding infers that BRAK-responsive cells are less likely to migrate to cancer tissue (see non-patent document 20). BRAK expression in adult mice is confirmed predominantly in the brain, lung, skeletal muscle and ovary (see non-patent document 14). There has been, however, no insight into behaviors and functions of BRAK-responsive cells in mouse individuals, in particular, behaviors and functions of monocytes and macrophages in white adipose tissue.

Accordingly, in order to reveal the relationship between chemotaxis of the monocytes and the macrophages to the white adipose tissue and BARK as well as to analyze dynamics of various parameters relating to obesity and insulin response, we prepared knockout non-human animals having the BRAK gene deleted, specifically BRAK knockout mice (BRAK-homo-deficient mice (-/-)). With this BRAK knockout mice and control mice from the same litter (wild-type mice (+/+), BRAK-hetero-deficient mice (+/-)), insulin resistance and the number of macrophages in the white adipose tissue were analyzed and compared (International Application No: PCT/JP2006/324622).

As a result, when BRAK knockout mice were fed on general diet, their weights and the amounts of white fat were significantly less than those of the control mice. When the mice were fed on high-fat diet, liver enlargement and fatty livers characteristic of obese mice were alleviated even though the amounts of white fat were similar to those of the control mice. In addition, the numbers of macrophages in the white adipose tissue of the BRAK knockout mice were significantly less compared to those of the control mice, and no insulin resistance associated with obesity was observed. Accordingly, we found that BRAK (CXCL14) was involved in process of obesity process due to food intake regulation, migration of macrophages to white adipose tissue, and acquirement of obese insulin resistance, a typical symptom of type 2 diabetes.

Non-patent Documents:
- 1.: Weisberg SP et al., J. Clin. Invest., 112:1796-1808 (2003)
- 2.: Xu H et al., J. Clin. Invest., 112:1821-1830 (2003)
- 3.: Wellen KE et al., J. Clin. Invest., 112:1785-1788 (2003)
- 4.: Wellen KE et al., J. Clin. Invest., 115:1111-1119 (2005)
- 5.: Uysal KT et al., Nature, 389:610-614 (1997)
- 6.: Hirosumi J et al., Nature, 420:333-336 (2002)
- 7.: Özcan U et al., Science, 306:457-461 (2004)
- 8.: Cai D et al., Nat. Med., 11:183-190 (2005)
- 9.: Arkan MC et al., Nat. Med., 11:191-198 (2005)
- 10.: Yamauchi T et al., Nat. Med., 7:941-946 (2001)
- 11.: Hromas R et al., Biochem. Biophys. Res. Commun., 255:703-706(1999)
- 12.: Frederick MJ et al., Am. J. Pathol., 156:1937-1950 (2000)
- 13.: Cao X et al., J. Immunol., 165:2588-2595 (2000)
- 14.: Sleeman MA et al., Int. Immunol., 12:677-689 (2000)
- 15.: Zlotnik A et al., Immunity, 12:121-127 (2000)
- 16.: Kurth I et al., J. Exp. Med., 194:855-861 (2001)
- 17.: Shellenberger TD et al., Cancer Res., 64:8262-8270 (2004)
- 18.: Schaerli P et al., Immunity, 23:331-342 (2005)
- 19.: Allinen M et al., Cancer Cell, 6:17-32 (2004)
- 20.: Shurin GV et al., J. Immunol., 174:5490-5498 (2005)

### DISCLOSURE OF THE INVENTION

Thus, the objectives of the present invention are to provide a compound that suppresses or inhibits BRAK (CXCL14) functions (e.g., production or action of BRAK) and use thereof, more particularly, to provide an antibody that recognizes BRAK and use thereof. Specifically, the objective of the invention is to provide effective use of a compound that suppresses or inhibits the BRAK functions (more particularly, an antibody that specifically recognizes BRAK) for diagnosing the risk of type 2 diabetes and for preventing and/or treating (alleviating) type 2 diabetes and obesity.

In order to solve the above problems, we have gone through keen examination and consequently developed a compound (more particularly, an antibody) capable of solving the problems above and use thereof (e.g., diagnosis, prevention and treatment of type 2 diabetes and obesity), thereby accomplishing the present invention.

Thus, the present invention relates to the followings.
(1) An antibody that recognizes a polypeptide comprising an amino acid sequence represented by SEQ ID NO: 1 or 2, a derivative thereof, or a partial peptide thereof.
   Examples of the antibodies of the invention include monoclonal antibodies, humanized or human antibodies, labeled antibodies and combinations of these antibodies.
   Examples of the antibodies of the invention also include antibodies that have activities of decreasing the number of macrophages in white adipose tissue and/or ameliorating insulin resistance.
(2) A hybridoma cell that produces the antibody according to (1) above.
(3) A method for producing the antibody according to (1) above, comprising: culturing the hybridoma cell according to (2) above *in vivo* or *in vitro;* and collecting the antibody according to (1) above from the body fluid or culture.
(4) A diagnostic agent comprising the antibody according to (1) above.
   The diagnostic agent of the invention may be used for diagnosing, for example, type 2 diabetes and/or obesity.
(5) A pharmaceutical agent comprising the antibody according to (1) above.
   The pharmaceutical agent of the invention may be used, for example, as an agent for preventing and/or treating type 2 diabetes and/or obesity, as an agent that decreases the number of macrophages in white adipose tissue, or as an agent that ameliorates insulin resistance.
(6) A method for preventing and/or treating type 2 diabetes and/or obesity, comprising administering an effective amount of the antibody according to (1) above to a mammal.
(7) A method for decreasing the number of macrophages in white adipose tissue, comprising administering an effective amount of the antibody according to (1) above to a mammal.
(8) A method for ameliorating insulin resistance, comprising administering an effective amount of the antibody according to (1) above to a mammal.
(9) Use of the antibody according to (1) above for producing an agent for diagnosing type 2 diabetes and/or obesity. In addition, an antibody according to (1) above for diagnosing type 2 diabetes and/or obesity.
(10) Use of the antibody according to (1) above for producing an agent for preventing and/or treating type 2 diabetes and/or obesity. In addition, an antibody according to (1) above for preventing and/or treating type 2 diabetes and/or obesity.
(11) Use of the antibody according to (1) above for producing an agent for decreasing the number of macrophages in white adipose tissue. In addition, an antibody according to (1) above for decreasing the number of macrophages in white adipose tissue.
(12) Use of the antibody according to (1) above for producing an agent for ameliorating insulin resistance. In addition, an antibody according to (1) above for ameliorating insulin resistance.

### BRIEF DESCRIPTION OF THE DRAWING

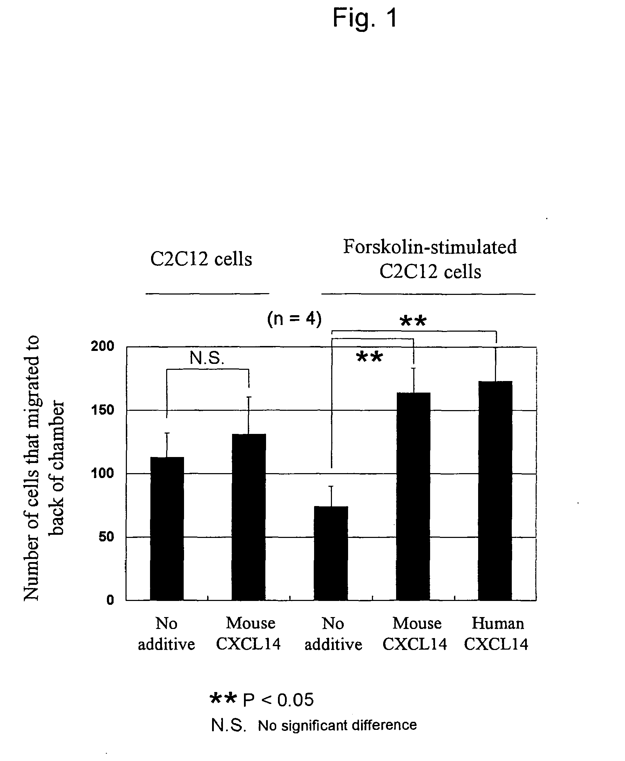

Figure 1 is data examining chemotaxis of C2C12 cells and forskolin-stimulated C2C12 cells to CXCL14. After six hours of culture, the number of cells that migrated from inside to the back of the Chemotaxicell microchamber was determined to test statistical significant difference. The graph shows average value ± standard error for each group.

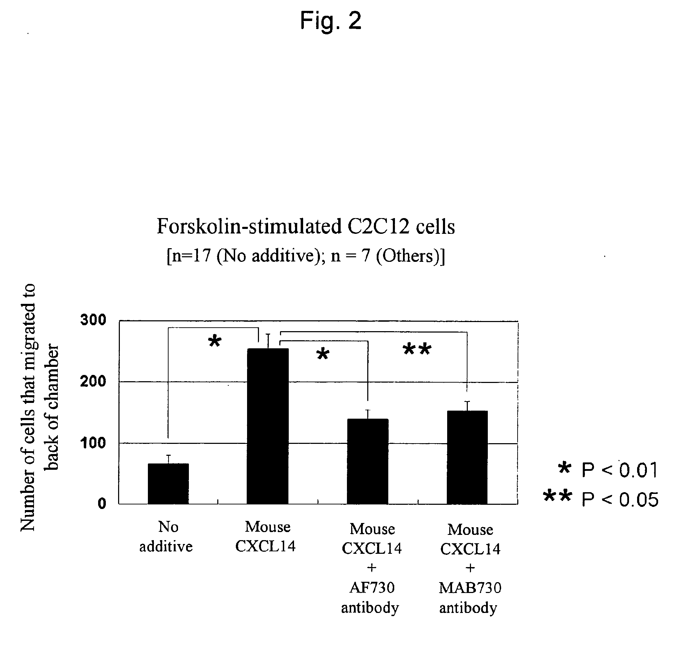

Figure 2 is data examining whether chemotaxis of forskolin-stimulated C2C12 cells to CXCL14 is neutralized by addition of an anti-mouse CXCL14-specific antibody. After six hours of culture, the number of cells that migrated from inside to the back of the microchamber was determined to test statistical significant difference. The graph shows average value ± standard error for each group.

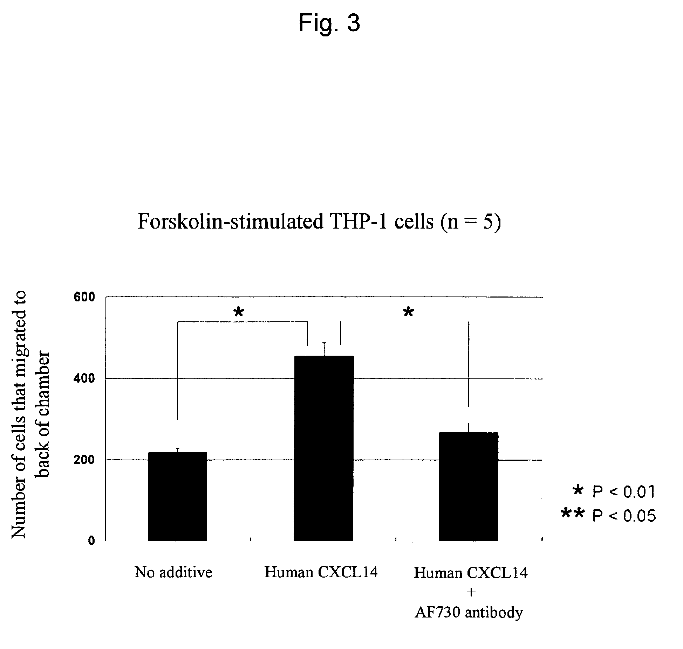

Figure 3 is data examining whether chemotaxis of forskolin-stimulated THP-1 cells to CXCL14 is neutralized by addition of an anti-mouse CXCL14-specific antibody. After two hours of culture, the number of cells that migrated from inside to the back of the Chemotaxicell microchamber was determined to test statistical significant difference. The graph shows average value ± standard error for each group.

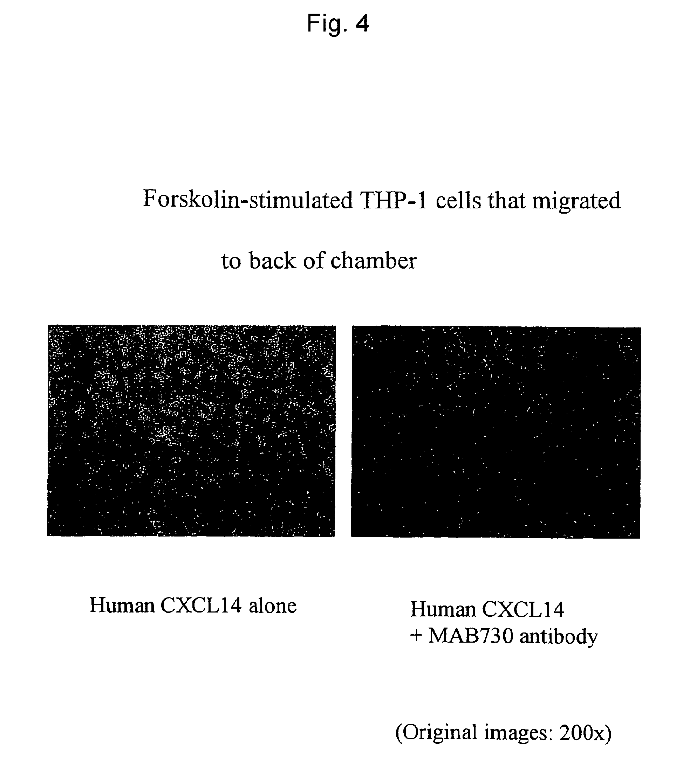

Figure 4 is data examining whether chemotaxis of forskolin-stimulated THP-1 cells to CXCL14 is affected by addition of an anti-mouse CXCL14 monoclonal antibody. Typical pictures of stained cells that migrated from inside to the back of the Chemotaxicell microchamber are shown.

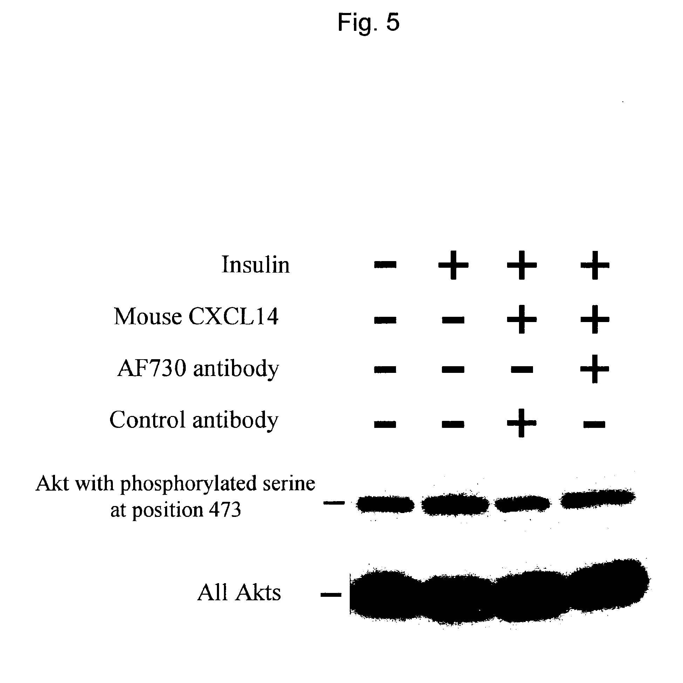

Figure 5 shows that an anti-mouse CXCL14-specific antibody neutralizes an activity of CXCL14 inhibiting insulin signaling. Differentiation-induced C2C12-derived myocytes were stimulated with insulin in the presence of indicated substances and phosphorylation of Akt Ser⁴⁷³ was determined by western blotting.

### BEST MODES FOR CARRYING OUT THE INVENTION

Hereinafter, the present invention will be described in more detail. The scope of the invention should not be limited to the description and the present invention may appropriately be varied and carried out without departing from the scope of the invention.

The present specification incorporates the entire content of the specification of Japanese Patent Application Publication No. 2007-030027 based on which the present application claims priority. All of the documents cited herein, for example, prior art documents, laid-open patent publications, patent publications and other patent documents are incorporated herein by reference.

Herein, BRAK and CXCL14 are synonymous and both terms are used in the same sense.

### 1. Summary of the Invention

### <Process of achieving the invention>

We noticed the increase of mRNA expression in the skeletal myocytes of muscular dystrophy model mice mdx and focused on physiological functions of BRAK (CXCL14).

Recently, macrophages have been reported successively to play an important role in the repair of skeletal muscle injury, liver injury or the like. Thus, we produced CXCL14(-/-) mdx and CXCL14(+/-) mdx mice by mating CXCL14 gene-deficient mice [CXCL14(-/-) mice] with mdx mice. Blood-Creatine kinase (CK) levels of these mice were determined regularly. Although fluctuation was present among the individuals, the CK levels of the CXCL14(-/-) mdx mice group were significantly lower than those of the CXCL14(+/-) mdx mice group. However, when the skeletal muscle and the diaphragm of aged CXCL14(-/-) mdx mice were histochemically analyzed, myodegeneration unique to mdx mice occurred equally likely to the aged CXCL14(+/-) mdx mice. In experiments of skeletal muscle injury with *habu* snake venom injection, CXCL14(-/-) mdx mice showed nearly equal kinetics of muscular tissue regeneration to that of the normal mice. Based on these facts, decrease of blood-CK levels of the CXCL14(-/-) mdx mice was seemingly not caused by alleviation of fascial degeneration but it rather seemed to reflect different physiology on the muscles of CXCL14.

Thus, the basic nature of the CXCL14(-/-) mdx mice was carefully compared, the average weight of six-month-old CXCL14(-/-) female mice fed on general diet was less than that of CXCL14(+/-) female mice by 25% and the amount of white fat around uterus decreased to one-fourth. This weight difference was caused by less food intake by the CXCL14(-/-) mice. Next, high-fat diet was given to these mice. The CXCL14(-/-) mice gradually become obese although they still weighed lighter than the CXCL14(+/- ) mice and the amounts of visceral white fat became nearly equal. Liver enlargement and fatty liver unique to obese mice were alleviated in these CXCL14(-/-) mice. Sugar levels of the CXCL14(-/-) mice fed on high-fat diet were significantly lower than those of the high-fat diet-fed CXCL14(+/-) mice. When the changes in sugar levels following insulin administration were measured, insulin resistance of the obese individuals was ameliorated for high-fat diet-fed CXCL14(-/-) mice. From these results, we decided to analyze in more detail about the physiological roles of CXCL14 in type 2 diabetes and obesity. Since the amelioration of insulin resistance of CXCL14(-/-) mice was particularly predominant in the skeletal muscle, the decrease in the CK values observed in the CXCL14(-/-) mdx mice appeared to be closely related to glucose metabolism.

### <Mechanism of Actions of the Present Invention and BRAK>

Based on the results from the analysis using BRAK (CXCL14) gene-deficient mouse strain [CXCL14(-/-) mice], we found out that CXCL14 was involved in the acquirement of obese insulin resistance, i.e., a typical symptom of type 2 diabetes, and the obesity process due to food intake regulation (these are described in more detail in (1) and (2) below). From these findings, we found that a compound that suppresses or inhibits the functions of CXCL14 (e.g., production or action of CXCL14), in particular an antibody that specifically recognizes CXCL14, was effective as a novel agent for treating type 2 diabetes and potentially as an anti-obesity agent. We further found that the above-mentioned compound (specifically, an anti-BRAK antibody) was also effective as a diagnostic agent for type 2 diabetes and obesity risk.

### (1) Role of CXCL14 in acquiring obese insulin resistance

CXCL14 is a protein with 77 amino acids (99 amino acids if a signal peptide is included) obtained by cloning chemokine cDNA that induces a tissue macrophage (Hromas, R. et al., BBRC, 255: 703-706, 1999; Frederick, M.J. et al., Am. J. Pathol., 156: 1937-1950, 2000; Sleeman, M.A. et al., Int. Immunol., 12: 677-689, 2000). We found that CXCL14(-/-) mice were less apt to become obese insulin resistant on high-fat diet. Based on this fact, we assumed that the above-described gene-modified mice (CXCL14(-/-) mice) were useful for research and development of a therapeutic agent for type 2 diabetes (International Application No: PCT/JP2006/324622).

CXCL14(+/-) mice were raised on high-fat diet for 12 weeks as control mice. As with general overeating obese mice, 1) macrophage infiltration into visceral white fat; 2) decrease in the blood concentration of adiponectin; 3) increase in the blood concentration of retinol binding protein 4 (RBP4), one of the causative factors of insulin resistance; 4) fatty liver; and 5) slowing in decrease in the blood-sugar levels upon abdominal administration of insulin, were observed in these mice. Meanwhile, 1) to 5) above were all significantly improved in CXCL14(-/-) mice fed on high-fat diet. This difference was particularly prominent in female mice. Double genetic mutant mice were produced by mating a transgenic mouse forcibly expressing CXCL14 in a skeletal muscle-specific manner with a CXCL14(-/-) mouse. Insulin resistance due to high-fat diet recovered in these double genetic mutant mice. On the other hand, CXCL14 partially inhibited Akt kinase phosphorylation and 2-deoxy-glucose (analogue of glucose) intake caused by insulin stimulation in an *in vitro* experimental system of insulin-stimulated myotubes prepared by inducing differentiation of C2C12 myoblast lines. These experimental results showed, for the first time, that CXCL14 not only acts as a macrophage chemotactic factor but also directly regulates sugar metabolism. Since insulin response was particularly improved in the skeleton muscle of the CXCL14(-/-) mice fed on high-fat diet, CXCL14 appears to not only induce chronic inflammatory reaction due to macrophage infiltration into visceral fat but also directly inhibit sugar absorption by the skeleton muscle, thereby contributing to development of obese insulin resistance.

### (2) Relationship between CXCL14 and obesity

The average weight of CXCL14(-/-) female mice was lighter than that of CXCL14(+/-) female mice by 20-25% in general diet conditions as well as high-fat diet conditions. The same was observed in double-mutant mice of genetically-obese leptin-deficient mice ob/ob and CXCL14(-/-) mice. While 8-10-week-old CXCL14(-/-) ob/ob female mice presented obvious obesity, their weights lightened by 15-20% as compared to CXCL14(-/-) ob/ob female mice and their food intake also decreased by approximately 20%. Thus, CXCL14 seemed to have a significant effect of increasing appetite or suppressing loss of appetite by a mechanism independent from leptin system.

### 2. Compound that Suppresses or Inhibits CXCL14 Functions

A compound that suppresses or inhibits CXCL14 functions may be, for example, a compound that suppresses or inhibits production or action of CXCL14. Examples of such compounds include (i) a compound that suppresses or inhibits the functions of the CXCL14 gene, (ii) a compound that suppresses or inhibits the CXCL14 functions by directly interacting (e.g., binding or assembling) with a CXCL14 molecule, and (iii) a compound that suppresses or inhibits the CXCL14 functions by interacting (e.g., binding or assembling) with a substance (a substrate of CXCL14) or a cell targeted by CXCL14. These compounds are not particularly limited and may be either a so-called low- or high-molecular compound or a compound having a molecular weight between them.

Specifically, examples of the compounds described in (i) above include nucleic acids and other various organic compounds that suppress or inhibit the functions of the CXCL14 gene. Such nucleic acids may be any one of polynucleotides, oligonucleotides (DNA, RNA) and peptide nucleic acids (PNA), specific types being, for example, antisense DNA, siRNA, shRNA and microRNA.

Examples of the compounds described in (ii) above include antibodies and enzymes that specifically recognize CXCL14, natural or synthetic polypeptides and oligopeptides not included in such antibodies or enzymes, and other various organic compounds.

The compounds of (iii) above may be referred to as compounds that indirectly suppress or inhibit CXCL14 since they do not act directly on CXCL14 and a gene thereof. Examples of such compounds include antibodies and enzymes that specifically recognize a substance targeted by CXCL14, natural or synthetic polypeptides and oligopeptides not included in such antibodies or enzymes, and other various organic compounds.

The compounds described above that suppress or inhibit the CXCL14 functions may preferably be used as a diagnostic agent for type 2 diabetes and obesity, an agent or a method for preventing and/or treating type 2 diabetes and obesity, an agent or a method for decreasing the number of macrophages in the white adipose tissue and an agent or a method for ameliorating insulin resistance.

Hereinafter, an "antibody that recognizes CXCL14", one of the particularly favorable embodiments among the various compounds mentioned above that suppress or inhibit the CXCL14 functions, will be described in detail including its use in various applications. Similar description may apply to other compounds as to the use in various applications.

### 3. Antibody of the Invention

SEQ ID NOS: 1-6 among the sequences identified herein represent the amino acid sequences of the following peptides. The amino acid sequences (99 amino acids) represented by SEQ ID NOS: 1 and 2 each have a signal sequence of 22 amino acids at the N terminus. The amino acid sequences represented by SEQ ID NOS: 1 and 2 have been assigned "Accession number: AAD03839" and "Accession number: AAD34157" on the website of NCBI (GenBank) (http://www.ncbi.nlm.nih.gov/), respectively. The amino acid sequences represented by SEQ ID NOS: 3-6 are each a part of the amino acid sequences represented by SEQ ID NOS: 1 and 2 while the numerical range in the parentheses indicates their positions (indicated as the number of amino acid residues counted from the N terminus) in the amino acid sequence represented by SEQ ID NOS: 1 and 2.
[SEQ ID NO: 1] human CXCL14
[SEQ ID NO: 2] mouse CXCL14
[SEQ ID NO: 3] human CXCL14 (23-99)
[SEQ ID NO: 4] human CXCL14 (24-35)
[SEQ ID NO: 5] mouse CXCL14 (23-99)
[SEQ ID NO: 6] mouse CXCL14 (24-35)

Below are the human CXCL14 amino acid sequence represented by SEQ ID NO: 1 and the mouse CXCL14 amino acid sequence represented by SEQ ID NO: 2 (both given in single letter codes for the amino acids). In these amino acid sequences, the underlined amino acid residues (total of two residues) are the difference between the human CXCL14 and the mouse CXCL14 in parts other than the signal sequence.

### <Human CXCL14 (SEQ NO: 1)>

MRLLAAALLLLLLALYTARVDGSKCKCSRKGPKIRYSDVKKLEMKPKY
PHCEEKMVIITTKSVSRYRGQEHCLHPKLQSTKRFIKWYNAWNEKRRVYEE

### <Mouse CXCL14 (SEQ ID NO: 2)>

MRLLAAALLLLLLALCASRVDGSKCKCSRKGPKIRYSDVKKLEMKPKY
PHCEEKMVIVTTKSMSRYRGQEHCLHPKLQSTKRFIKWYNAWNEKRRVYEE

Proteins (polypeptides or partial peptides) mentioned in this specification have the N terminus (amino terminus) on the left side and the C terminus (carboxyl terminus) on the right side in accordance with the custom of peptide representation. The proteins used with the invention such as polypeptides comprising the amino acid sequence represented by SEQ ID NO: 1 may have any of a carboxyl group, carboxylate, amide or ester at the C terminus.

Examples of derivatives of polypeptides recognizable by the antibody of the invention, namely human or mouse CXCL14 derivatives, include those in which the amino acid residues of the amino acid sequence represented by SEQ ID NO: 1 or 2 are partially substituted with a replaceable group(s), partially deleted, or partially added or inserted with an amino acid residue(s). Examples of derivatives of polypeptides having the amino acid sequence represented by SEQ ID NO: 1 or 2 include those having one or more amino acids (preferably, approximately 1-10, more preferably several (1-5) and still more preferably 1, 2 or 3 amino acids) deleted, those having one or more amino acids (preferably, approximately 1-20 amino acids, more preferably approximately 1-10, more preferably several (1-5) and still more preferably 1, 2 or 3 amino acids) added to the amino acid sequence above, those having one or more amino acids (preferably, approximately 1-20, more preferably approximately 1-10, more preferably several (1-5) and still more preferably 1, 2 or 3 amino acids) inserted into the amino acid sequence above, or those having one or more amino acids (preferably, approximately 1-10, more preferably several (1-5) and still more preferably 1, 2 or 3 amino acids) substituted with other amino acids.

The human or mouse CXCL14 derivatives described above may be used, for example, as salts (preferably physiologically acceptable acid addition salts) formed with physiologically acceptable acids (e.g., inorganic and organic acids) or bases (e.g., alkali metal salts), examples including salts formed with inorganic acids (e.g., hydrochloric acid, phosphoric acid, hydrobromic acid and sulfuric acid) and salts formed with organic acids (e.g., acetic acid, formic acid, propionic acid, fumaric acid, maleic acid, succinic acid, tartaric acid, citric acid, malic acid, oxalic acid, benzoic acid, methanesulfonic acid and benzenesulfonic acid).

Examples of partial peptides recognizable by the antibody of the invention, namely partial peptides of human or mouse CXCL14 or derivatives thereof, include polypeptides having the amino acid residues partially deleted, partially substituted with a replaceable group(s) (e.g., Cys, hydroxyl group, etc.), or partially deleted and partially substituted with a replaceable group(s) (e.g., Cys, hydroxyl group, etc.) in the amino acid sequence represented by SEQ ID NO: 1 or 2.

Examples of such partial peptides include those having approximately 22 residues (signal peptide portion) at the N-terminus of the human or mouse CXCL14 or derivatives thereof deleted. More specifically, such partial peptides are preferably (i) a polypeptide having the amino acids 23-99 (SEQ ID NO: 3) of the amino acid sequence represented by SEQ ID NO: 1; (ii) a polypeptide having the amino acids 24-35 (SEQ ID NO: 4) of the amino acid sequence represented by SEQ ID NO: 1; (iii) those having a part (e.g., one) of the amino acid residues of these polypeptides of (i) and (ii) substituted with replaceable group(s); (iv) a polypeptide having the amino acids 23-99 (SEQ ID NO: 5) of the amino acid sequence represented by SEQ ID NO: 2; (v) a polypeptide having the amino acids 24-35 (SEQ ID NO: 6) of the amino acid sequence represented by SEQ ID NO: 2; and (vi) those having a part (e.g., one) of the amino acid residues of these polypeptides of (iv) and (v) substituted with a replaceable group(s).

A method for preparing an antigen to an antibody and a method for producing an antibody may be a known method such as a method described in WO 94/17197 or a method based thereon. Hereinafter, an exemplary method will be described.

### (1) Preparation of antigen

An antigen used for preparing an antibody of the invention may be, for example, CXCL14 (BRAK), a derivative thereof, a partial peptide thereof, or a synthetic peptide having one or more types of antigen determinants identical to those of CXCL14 (hereinafter, these are simply referred to as "CXCL14 antigens"). CXCL14, a derivative thereof or a partial peptide thereof may be those described above. A CXCL14 antigen may be prepared from a mammal such as human, mouse, possibly monkey, rat or swine by a known method or a method based thereon. Alternatively, a CXCL14 antigen used may be a commercially available natural purified preparation or a synthetic peptide.

A CXCL14 antigen may be prepared by a known method such as a method described in WO02/06483. A CXCL14 antigen may also be prepared (a) by a known method or a method based thereon from tissue or a cell from a mammal such as human, mouse, possibly monkey, rat or swine; (b) by chemical synthesis according to a known peptide synthesis method with a peptide synthesizer or the like; or (c) by culturing a transformant containing DNA coding for CXCL14 or a derivative thereof.
(a) Where a CXCL14 antigen is prepared from mammal tissue or cell, the tissue or the cell is homogenized, extracted with an acid or an alcohol, and the extract is purified and isolated by a combination of chromatographies such as salting-out, dialysis, gel filtration, reversed-phase chromatography, ion-exchange chromatography or affinity chromatography, thereby preparing a CXCL14 antigen.
(b) A synthetic peptide used for chemically synthesizing a CXCL14 antigen may be, for example, one having the same structure as a native purified CXCL14 antigen, or a peptide containing one or more types of amino acid sequences identical to amino acid sequences having 3 or more, preferably 6 or more amino acids of the amino acid sequence of CXCL14.
(c) Where a CXCL14 antigen is produced with a transformant containing DNA, the DNA may be prepared according to a known cloning method (e.g., a method described in Molecular Cloning (2nd ed.; J. Sambrook et al., Cold Spring Harbor Lab. Press, 1989)). Examples of such cloning method include: (1) a method for obtaining a transformant containing DNA coding for a CXCL14 antigen from a cDNA library using a DNA probe or DNA primers designed based on the amino acid sequence of the CXCL14 antigen by hybridization; or (2) a method for obtaining a transformant containing DNA coding for a CXCL14 antigen by PCR using DNA primers designed based on an amino acid sequence of the CXCL14 antigen.

A partial peptide obtained by CXCL14 hydrolysis may be, for example, a partial peptide or a mixture thereof obtained by sequentially hydrolyzing a polypeptide having the amino acid sequence represented by SEQ ID NO: 1 or 2 from the N- and/or the C-terminus with exoprotease such as aminopeptidase or carboxypeptidase, or a partial peptide or a mixture thereof obtained by hydrolyzing a polypeptide having the amino acid sequence represented by SEQ ID NO: 1 or 2 with various endopeptidases.

The synthetic peptide may be produced by a known routine procedure which may be either solid- or liquid-phase synthesis. Specifically, a partial peptide or amino acids that may constitute this peptide and the remaining part are condensed and a protecting group of the product, if any, is detached, thereby producing a peptide of interest. Such a condensation method and a detachment method of the protecting group are, for example, described in B. Merrifield [Journal of American Chemical Society (J. Am. Chem. Soc.), 85, 2149, 1963], M. Bodanszky and M.A. Ondetti [Peptide Synthesis, Interscience publishers, New York 1966], Schroeder and Luebke [The Peptide, Academic Press, New York, 1965], Nobuo Izumiya et al. [Fundamentals and Experiments of Peptide Synthesis, Maruzen, 1985], and Haruaki Yajima and Shunpei Yanagihara [Seikagaku Jikken Kouza 1: Tanpakusitu no Kagaku IV (Course for Biochemical Experiments 1: Chemistry of Protein IV), 205, 1977]. After the reaction, the peptide may be purified by combining general purification methods such as solvent extraction, distillation, column chromatography, liquid chromatography, recrystallization or the like. If the peptide obtained by the above method is a free body, it may be transformed into an appropriate salt by a known method whereas if the peptide is obtained as a salt, it may be transformed into a free body by a known method.

An amide body of the peptide may be obtained by using a commercially available resin for peptide synthesis suitable for amide formation. Examples of such resins include a chloromethyl resin, a hydroxymethyl resin, a benzhydrylamine resin, an aminomethyl resin, a 4-benzyloxybenzyl alcohol resin, a 4-methylbenzhydrylamine resin, a PAM resin, a 4-hydroxymethyl methylphenyl acetamidemethyl resin, a polyacrylamide resin, a 4-(2',4'-dimetoxylphenyl-hydroxymethyl)phenoxy resin, and a 4-(2',4'-dimetoxylphenyl-Fmoc aminoethyl)phenoxy resin. With the use of such a resin, amino acids having an alpha-amino group and a side-chain functional group appropriately protected are condensed on the resin following the sequence of the peptide of interest according to one of various condensation methods. At the end of the reaction, each protecting group is removed upon peptide cleavage from the resin. Alternatively, a peptide of interest may also be obtained by taking out a part of the protected peptide with a chlorotrityl resin, an oxime resin, 4-hydroxybenzoic acid resin or the like, and further removing the protecting group by a routine procedure.

Various activating reagents available for peptide synthesis may be used for the condensation of the protected amino acids, although carbodiimides are preferably used. Examples of such carbodiimides include DCC, N,N'-diisopropylcarbodiimide and N-ethyl-N'-(3-dimethylaminoprolyl)carbodiimide. For activation with each activating reagent, the protected amino acid may be directly added to the resin with a racemization suppressant (e.g., HOBt, HOOBt, etc.) or an activated amino acid that has been protected beforehand as a symmetric anhydride, HOBt ester or HOOBt ester may be added to the resin. A solvent used for the activation of the protected amino acid and the condensation with the resin may be appropriately selected from known solvents available for peptide condensation reaction. Examples of such solvents used for activation of protected amino acids or condensation with a resin include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethylsulfoxide, tertiary amines such as pyridine, ethers such as dioxane and tetrahydrofuran, nitriles such as acetonitrile and propionitrile, esters such as methyl acetate and ethyl acetate, and an appropriate mixture thereof. A reaction temperature is appropriately selected from a range known for peptide binding reaction, generally selected from a range of about -20°C to about 50°C. The activated amino acid derivatives are usually used in an about 1.5- to about 4-fold excess. If the condensation is inadequate when tested by ninhydrin reaction, the condensation reaction may be repeated without removing the protecting groups so as to achieve adequate condensation. When the condensation is inadequate even after repetitive reactions, the unreacted amino acids are acetylated with acetic anhydride or acetylimidazole so as not to affect the subsequent reactions.

Examples of the protecting groups for amino groups of the raw amino acids include a Z group, a Boc group, a tertiary pentyloxycarbonyl group, an isobornyloxycarbonyl group, a 4-methoxybenzyloxycarbonyl group, a Cl-Z group, a Br-Z group, an adamantyloxycarbonyl group, a trifluoroacetyl group, a phthaloyl group, a formyl group, a 2-nitrophenylsulfenyl group, a diphenylphosphinothioyl group and a Fmoc group. Examples of protecting groups of carboxy groups include a C₁₋₆ alkyl group, a C₃₋₈ cycloalkyl group and C₇₋₁₄ aralkyl group as well as a 2-adamantyl group, a 4-nitrobenzyl group, a 4-methoxybenzyl group, a 4-chlorobenzyl group, a phenacyl group and a benzyloxycarbonyl hydrazide group, a tertiary butoxycarbonyl hydrazide group and a trityl hydrazide group.

A hydroxyl group for serine or threonine may be protected, for example, by esterification or etherification. Examples of groups suitable for this esterification include lower (C₁₋₆) alkanoyl groups such as an acetyl group, aroyl groups such as a benzoyl group and carbon-derived groups such as a benzyloxycarbonyl group and an ethoxycarbonyl group. Groups suitable for etherification are, for example, a benzyl group, a tetrahydropyranyl group and a tertiary butyl group.

Examples of protecting groups for a phenolic hydroxyl group of tyrosine include a Bzl group, a Cl-Bzl group, a 2-nitrobenzyl group, a Br-Z group and a tertiary butyl group.

Examples of protecting groups for imidazole groups of histidine include a Tos group, a 4-methoxy-2,3,6-trimethylbenzenesulfonyl group, a DNP group, a Bom group, a Bum group, a Boc group, a Trt group and a Fmoc group.

Examples of a raw material with an activated carboxyl group include corresponding acid anhydride, azide and active esters [esters with alcohols (e.g., pentachlorophenol, 2,4,5-trichlorophenol, 2,4-dinitrophenol, cyanomethyl alcohol, para-nitrophenol, HONB, N-hydroxysuccinimide, N-hydroxyphthalimide and HOBt)]. An example of a raw material with an activated amino group includes corresponding amide phosphate.

Examples of a method for removing (detaching) a protecting group include a catalytic reduction method using a hydrogen stream in the presence of a catalyst such as Pd-black or Pd-carbon, an acid treatment with anhydrous hydrogen fluoride, methanesulfonic acid, trifluoromethanesulfonic acid, trifluoroacetic acid, a mixture thereof or the like, a base treatment with diisopropylethylamine, triethylamine, piperidine, piperazine or the like, and reduction with sodium in liquid ammonia. Detachment reaction by an acid treatment mentioned above is generally carried out at a temperature of -20°C to 40°C while addition of a cation capture agent such as anisole, phenol, thioanisole, meta-cresol, para-cresol, dimethyl sulfide, 1,4-butanedithiol or 1,2-ethanedithiol is effective. Moreover, a 2,4-dinitrophenyl group used as an imidazole-protecting group of histidine is removed by a thiophenol treatment while a formyl group used as an indole-protecting group for tryptophan may be removed not only by deprotection by an acid treatment in the presence of 1,2-ethanedithiol, 1,4-butanedithiole or the like mentioned above but also by an alkali treatment with diluted sodium hydrate, diluted ammonia or the like.

Protection and protecting groups for functional groups that should not be involved with the reaction of the raw material, detachment of these protecting groups, and activation of functional groups involved in the reaction may be appropriately selected from known groups or known procedures.

Another method for obtaining an amide body of a peptide includes: first, amidating the alpha-carboxyl group of the carboxy-terminal amino acid; then extending a peptide chain to an intended length from the amino group side; producing a peptide having the protecting group removed only for the alpha-amino group at the N-terminus of the peptide chain as well as a peptide (or an amino acid) having the protecting group removed only for the carboxyl group at the C-terminus; and condensing these peptides in a mixed solvent as described above. Details on condensation reaction are the same as described above. After purifying the protected peptide resulting from condensation, all of the protecting groups are removed by the method described above, thereby obtaining crude peptide of interest. This crude peptide may be purified by any of the known various purification procedures and the main fraction may be freeze-dried, thereby obtaining an amide body of the peptide of interest.

In order to obtain an ester body of a peptide, an alpha-carboxyl group of a carboxy-terminal amino acid is condensed with a suitable alcohol to give an amino acid ester, with which an ester body of the peptide of interest can be obtained in the same manner as the amide body of the peptide.

A CXCL14 antigen that has been insolubilized may be immunized directly. Alternatively, a complex of a CXCL14 antigen binding to or adsorbed onto an appropriate carrier may be immunized. A mix ratio of the carrier and the CXCL14 antigen (hapten) may be any ratio as long as an antibody is efficiently produced against the CXCL14 antigen bound to or adsorbed onto the carrier. Usually, a polymeric carrier commonly used for producing an antibody to the hapten is used in 0.1-100 parts to a part of hapten. Such a polymeric carrier may be, for example, a natural polymeric carrier or a synthetic polymeric carrier. Examples of natural polymeric carriers include serum albumin derived from a mammal such as bovine, rabbit, human or the like, thyroglobulin from a mammal such as bovine, rabbit or the like, hemoglobin from a mammal such as bovine, rabbit, human, sheep or the like, and KHL (keyhole limpet) hemocyanin. Examples of synthetic polymeric carriers include various latexes like polymers or copolymers such as polyamino acids, polystyrenes, polyacryls, polyvinyls and polypropylenes.

Various condensation agents may be used for coupling the hapten and the carrier. Examples of conveniently used condensation agents include diazonium compounds such as bis-diazotized benzidine that cross-links tyrosine, histidine and tryptophan, dialdehyde compounds such as glutaraldehyde that cross-links amino groups with each other, diisocyanate compounds such as toluene-2,4-diisocyanate, dimaleimide compounds such as N,N'-o-phenylenedimaleimide that cross-links thiol groups with each other, a maleimide active ester compound that cross-links an amino group and a thiol group, and a carbodiimide compounds that cross-links an amino group and a carboxyl group. Alternatively, in order to cross-link amino groups with each other, an active ester reagent (e.g., SPDP, etc.) having a dithiopyridyl group is reacted with one amino group and subsequently reduced to introduce a thiol group while a maleimide group is introduced into the other amino group with a maleimide active ester reagent, and then both amino groups are reacted.

### (2) Preparation of monoclonal antibody

Although an antibody of the invention is not particularly limited and it may be either a polyclonal antibody or a monoclonal antibody, it is preferably a monoclonal antibody. Hereinafter, a method for preparing an antibody of the invention will be described taking the case of a monoclonal antibody as an example.

A CXCL14 antigen is administered to a warm-blooded animal, for example, through intraperitoneal injection, intravenous injection, subcutaneous injection or the like, alone or together with a carrier and a diluent, to a site that allows antibody production. In order to enhance the antibody production upon administration, a complete Freund's adjuvant or an incomplete Freund's adjuvant may be administered. Administration is usually performed once in every 2-6 weeks for a total of about 2-10 times. Although examples of warm-blooded animals include monkey, rabbit, dog, guinea pig, mouse, rat, sheep, goat and chicken, a mouse is preferably used for a production of an antibody, especially a monoclonal antibody.

For production of a monoclonal antibody, individuals with positive antibody titers are selected from the CXCL14 antigen-immunized warm-blooded animals such as mice. Two to five days following the final immunization, spleens or lymph nodes are collected from these individuals to fuse antibody-producing cells contained therein with myeloma cells, thereby preparing anti-CXCL14 monoclonal antibody-producing hybridomas. The anti-CXCL14 antibody titer in a serum may be measured, for example, by allowing a labeled CXCL14 described below to react with an antiserum and determining the activity of the labeling agent bound to the antibody. The fusion procedure may be carried out by a known method such as Kohler and Milstein [Nature, 256, 495 (1975)]. Examples of fusion accelerators include polyethyleneglycol (PEG) and Sendai virus, while PEG is preferably used. Examples of myeloma cells include NS-1, P3U1, SP2/0 and AP-1, while P3U1 is preferably used. A favorable ratio of the number of the antibody-producing cells (spleen cells) used to the number of bone marrow cells is generally about 1:1 1 to 20:1 1 where PEG (preferably PEG1000 to PEG6000) is added at a concentration of about 10-80% and incubated generally at 20-40°C, preferably 30-37°C, generally for 1-10 minutes for carrying out efficient cell fusion.

Various methods may be employed for screening an anti-CXCL14 antibody-producing hybridoma. In an exemplary method, a hybridoma culture supernatant is added directly or with a carrier to CXCL14, a derivative thereof or a partial peptide thereof adsorbed onto a solid phase (e.g., a microplate) and then an anti-immunoglobulin antibody (an anti-mouse immunoglobulin antibody is used if mouse cells are used for the cell fusion) labeled with a radioactive substance or an enzyme or protein A is added to detect anti-CXCL14 monoclonal antibodies bound to the solid phase. In another exemplary method, a hybridoma culture supernatant and then CXCL14 labeled with a radioactive substance or an enzyme is added to an anti-immunoglobulin antibody or protein A adsorbed onto a solid phase to detect CXCL14 monoclonal antibodies bound on the solid phase. Screening and production of the anti-CXCL14 monoclonal antibodies are usually performed by adding HAT (hypoxanthine, aminopterin and thymidine) in a medium for animal cells (e.g., RPMI1640) containing 10-20% fetal bovine serum. An antibody titer of a hybridoma culture supernatant may be measured in the same manner as the above-described measurement for an anti-CXCL14 antibody titer in an antiserum.

Similar to usual separation and purification of a polyclonal antibody, separation and purification of an anti-CXCL14 monoclonal antibody is carried out by following a method for separating and purifying an immunoglobulin (e.g., salting-out method, alcohol precipitation method, isoelectric precipitation method, electrophoresis, adsorption-desorption on an ion exchanger (e.g., DEAE), ultracentrifugation method, gel filtration method, or specific purification in which the antibody is collected with an antigen-binding solid phase or an active adsorbent such as protein A or protein G followed by dissociation of the binding).

Furthermore, sorting between a hybridoma producing an anti-CXCL14 antibody that reacts with a partial region of CXCL14 and a hybridoma producing an anti-CXCL14 monoclonal antibody that reacts with CXCL14 but not with the said partial region may be carried out, for example, by determining the binding capacity between the peptide corresponding to that partial region and the antibody produced by the hybridoma.

Hence, an antibody of the invention may be produced by culturing a hybridoma cell *in vivo* in a warm-blooded animal or *in vitro* and collecting an antibody from its fluid or culture.

The resulting antibody of the invention may be used as an agent for diagnosing type 2 diabetes and obesity, an agent for preventing and/or treating type 2 diabetes and obesity, an agent that decreases the number of macrophages in white adipose tissue, or an agent that ameliorates insulin resistance.

For these purposes, the antibody of the invention may be used as an antibody molecule itself or as a fragment of the antibody or a single-chain antibody of the V region, and all of them are within the scope of the antibody of the invention. A fragment of the antibody refers to a partial region of the antibody, specific examples being F(ab')₂, Fab', Fab, Fv (variable fragment of antibody), sFv, dsFv (disulphide stabilized Fv) and dAb (single region antibody). A single-chain antibody of the V region comprises V_{L} (light-chain variable region) and V_{H} (heavy-chain variable region) combined by a linker.

A preferable embodiment of an antibody of the invention comprises a humanized antibody or a human antibody. These humanized and human antibodies may collectively be referred to herein as "human antibodies" or as "human monoclonal antibodies" in the case of monoclonal antibodies. Similar to general preparation of a monoclonal antibody, these antibodies may be prepared by immunizing a mammal having its immune system replaced with a human immune system.

In order to prepare a humanized antibody, a complementarity determining region (CDR) is grafted from a variable region of an antibody of a mammal (non-human animal) such as a mouse into a human variable region to restructure a variable region in which a human-derived framework region (FR) and a non-human animal-derived CDR are used. Then, this restructured variable region is linked to a human constant region, thereby preparing a humanized antibody. A humanized antibody may also be prepared as a chimeric antibody comprising a variable region from a non-human antibody and a constant region from a human antibody. Preparation of a humanized antibody is well known in the art.

Generally, specificity and binding affinity of an antigen-binding part of the V region, i.e., a hypervariable region, matter for a human antibody, but structurally it may be prepared from any animal. On the other hand, structures of other parts of the V region and the constant region preferably have the same structure as those of a human antibody. A genetic engineering method for preparing a genetic sequence common to human has been established.

### 4. Applications of Antibody of the Invention

Hereinafter, applications of an antibody of the invention will be described in detail.

### (1) A diagnostic agent comprising an antibody of the invention

An antibody (particularly, a human monoclonal antibody) of the invention may be used as an agent for diagnosing a disease associated with CXCL14 (BRAK), namely type 2 diabetes and/or obesity.

Specifically, an antibody of the invention may be used for the purpose of diagnosing the risk of type 2 diabetes and/or obesity. As described above, based on the results from the analysis with CXCL14-homo-deficient mice, CXCL14 was found to be involved in an acquirement of obese insulin resistance (a typical symptom of type 2 diabetes) and a process of obesity due to food intake regulation. Thus, an antigen level (CXCL14 level) in a biological sample such as a fluid, tissue or the like collected from a test subject (a measured solution or a test solution) is measured to assess the risk of the test subject to develop type 2 diabetes or obesity.

Measurement using the antibody of the invention is not particularly limited. Any measurement method may be employed as long as the amount of the antibody, the antigen or the antibody-antigen complex corresponding to the antigen level (CXCL14 level) in the measured solution is detected by chemical or physical means, and calculated with a standard solution containing a known level of antigen. Examples of such measurement methods include a sandwich assay, a competitive assay, an immunometric assay and nefelometry, while a sandwich assay or a competitive assay described below is preferable, particularly a sandwich assay, in terms of sensitivity and specificity.

### (A) Sandwich assay

A sandwich assay is a method for quantifying CXCL14 or a derivative thereof in a test solution by allowing reaction of an antibody of the invention insolubilized on a carrier (a solid-phased antibody), a labeled antibody of the invention (antibody with different epitope from that of the solid-phased antibody: labeled antibody) and a test solution, and then determining the activity of the labeling agent.

According to a sandwich assay, a test solution is allowed to react with an antibody of the invention insolubilized on a carrier (primary reaction), followed by reaction with a labeled antibody of the invention (secondary reaction). Subsequently, the activity of the labeling agent on the insolubilized carrier is determined to quantify the CXCL 14 level in the test solution. The primary and secondary reactions may be conducted simultaneously or at different times. The labeling agent and the insolubilization method may be similar to those described previously. Moreover, in an immunoassay employing a sandwich assay, an antibody for a solid phase and an antibody for labeling are not necessarily limited to a single type, and a mixture of two or more types of antibodies may be used for the purpose of enhancing the measurement sensitivity or the like. For CXCL14 measurement by a sandwich assay, for example, when the antibody used for the primary reaction recognizes a partial peptide on the C-terminus of CXCL14 or a derivative thereof, the antibody used for the secondary reaction is preferably an antibody that recognizes a part other than the said partial peptide at the C-terminus (i.e., N-terminus). When the antibody used for the primary reaction recognizes a partial peptide on the N-terminus of CXCL14 or a derivative thereof, the antibody used for the secondary reaction is preferably an antibody that recognizes a part other than the said partial peptide at the N-terminus (i.e., C-terminus). The labeled antibody is preferably labeled with horseradish peroxidase (HRP).

### (B) Competitive assay

A competitive assay is a method for quantifying CXCL14 or a derivative thereof in a test solution by competitively reacting the antibody of the invention, a test solution and a labeled CXCL14 or a derivative thereof, and determining the proportion of the labeled CXCL14 or the derivative thereof bound to the antibody. Quantification of CXCL14 or a derivative thereof in the test solution by a competitive assay is preferably carried out by using, for example, a solid phase technique. A specific example of a solid phase technique includes a method comprising: using an anti-mouse IgG antibody (from ICN/CAPPEL) as a solid-phased antibody; adding (i) an antibody of the invention, (ii) a peptide represented by SEQ ID NO: 1 or 2 labeled with HRP and (iii) a test solution to the plate having the solid-phased antibody thereon; and, following reaction, determining the activity of HRP adsorbed onto the solid phase, thereby quantifying CXCL14 or a derivative thereof.

### (C) Immunometric assay

According to an immunometric assay, an antigen in a test solution and a solid-phased antigen are allowed to competitively react with a certain amount of a labeled antibody of the invention, followed by separation between the solid phase and the liquid phase. Alternatively, an antigen in a test solution is allowed to react with an excess amount of a labeled antibody of the invention, then a solid-phased antigen is added to bind the unreacted labeled antibody of the invention to the solid phase, followed by separation between the solid phase and the liquid phase. Subsequently, the amount of the label in either of the phases is determined to quantify the antigen level in the test solution.

### (D) Nefelometry

According to nefelometry, the amount of insoluble precipitation resulting from antigen-antibody reaction in a gel or a solution is measured. When the antigen level in the test solution is low and the amount of precipitation is small, laser nefelometry that utilizes laser scattering is preferably used.

In the measurement methods of (A) to (D) above, a labeling agent used for the methods using a labeling substance is, but not limited to, a radioisotope, an enzyme, a fluorescent substance, a luminescent substance or the like. Preferable examples of radioisotopes include, but not limited to, [¹²⁵I], [¹³¹I], [³H] and [¹⁴C]. The above-mentioned enzymes are preferably, but not limited to, those that are stable and that have high specific activity, examples being beta-galactosidase, beta-glucosidase, alkaline phosphatase, peroxidase and malate dehydrogenase. Examples of the above-mentioned fluorescent substance include, but not limited to, fluorescamine and fluorescein isothiocyanate. Examples of the above-mentioned luminescent substance include, but not limited to, luminol, luminol derivatives, luciferin and lucigenin. For binding between the antigen and the labeling agent, a biotin-avidin compound may be used.

For insolubilization of an antigen or an antibody, physical adsorption may be employed, or alternatively a method employing chemical adsorption generally used for insolubilization or immobilization of a protein, an enzyme or the like may be used. Examples of carriers include insoluble polysaccharides such as agarose, dextran and cellulose, synthetic resins such as polystyrene, polyacrylamide and silicon, and glass.

No particular condition or manipulation is required upon applying each of these immunological measurements to the present invention. A system for measuring CXCL14 or a derivative thereof may be established by employing general conditions and manipulation for each method while taking technical considerations by those skilled in the art into account. For more information on the general technological means, reference may be made to reviews, text books or the like (e.g., see Hiroshi Irie "Radioimmunoassay", Kodansha, 1974; Hiroshi Irie "The Sequel to Radioimmunoassay", Kodansha, 1979; Eiji Ishikawa et al. (ed.), "Enzyme Immunoassay", Igaku-Shoin, 1978; Eiji Ishikawa et al. (ed.), "Enzyme Immunoassay (2nd ed.)", Igaku-Shoin, 1982; Eiji Ishikawa et al. (ed.), "Enzyme Immunoassay (3rd ed.)", Igaku-Shoin, 1987; "Methods in ENZYMOLOGY Vol. 70", Immunochemical Techniques (Part A); "Methods in ENZYMOLOGY Vol. 73", Immunochemical Techniques (Part B); "Methods in ENZYMOLOGY Vol. 74", Immunochemical Techniques (Part C); "Methods in ENZYMOLOGY Vol. 84", Immunochemical Techniques (Part D: Selected Immunoassays); "Methods in ENZYMOLOGY Vol. 92", Immunochemical Techniques (Part E: Monoclonal Antibodies and General Immunoassay Methods); "Methods in ENZYMOLOGY Vol. 121", Immunochemical Techniques (Part I: Hybridoma Technology and Monoclonal Antibodies) (all published from Academic Press)).

The antibody of the invention may also be used for preparing an antibody column for purifying CXCL14 or a derivative thereof, for detecting CXCL14 or a derivative thereof in each fraction upon purification, for analyzing behavior of CXCL14 or a derivative thereof in a test cell or the like.

### (2) Pharmaceutical agent comprising antibody of the invention

An antibody of the invention (particularly a human monoclonal antibody) may be used as an active component of a pharmaceutical agent such as a prophylactic/therapeutic agent for diseases associated with CXCL14 (BRAK), i.e., type 2 diabetes and/or obesity. Preferably, examples of such pharmaceutical agents further include an agent for decreasing macrophages in white adipose tissue and an agent for ameliorating insulin resistance.

Hereinafter, use of a pharmaceutical agent as such prophylactic/therapeutic agent will be described. Similar description may apply to use of such decreasing and ameliorating agents mentioned above.

The prophylactic/therapeutic agent comprising an antibody of the invention have low toxicity and may be parenterally or orally administered to a human or a mammal (e.g., mouse) directly as a liquid agent or as a pharmaceutical composition in an appropriate formulation. An antibody of the invention may be administered by itself or as an appropriate pharmaceutical composition. A pharmaceutical composition used for administration may comprise an antibody of the invention or a salt thereof with a pharmacologically-acceptable carrier, diluent or excipient. Such a pharmaceutical composition may be provided as a formation suitable for oral or parenteral administration. A parenteral composition may be, for example, an injection, a suppository or the or like, where the injection may comprise a formulation such as an intravenous injection, a subcutaneous injection, an intradermal injection, an intramuscular injection, a drip injection or the like. Such injections may be prepared according to a known method. According to an exemplary method, an injection may be prepared by dissolving, suspending or emulsifying the antibody of the invention or a salt thereof in a sterile aqueous or oily solution generally used for an injection. Examples of injectable aqueous solutions include isotonic solutions containing physiological saline, glucose, or other adjuvants, which may be used together with an appropriate solubilizing agent such as an alcohol (e.g., ethanol), a polyalcohol (e.g., propylene glycol, polyethylene glycol), a nonionic surfactant (e.g., Polysorbate 80, HCO-50 (polyoxyethylene (50 mol) adduct of hydrogenated castor oil)) or the like. Examples of oily solutions include a sesame oil, a soybean oil and the like while benzyl benzoate, benzyl alcohol or the like may be used together as a solubilizing agent. The prepared injection is preferably packed into an appropriate ampoule. A suppository used for rectal administration may be prepared by mixing the antibody above or a salt thereof with a general suppository base.

Examples of compositions for oral administration include solid or liquid formulations, specifically, tablets (including sugar-coated tablets and film-coated tablets), pills, granule, powder, capsules (including soft capsules), syrups, emulsions, suspensions and the like. Such compositions may be produced according to a known method and may contain a carrier, a diluent or an excipient generally used in the art. A carrier or an excipient used for a tablet may be, for example, lactose, starch, sucrose and magnesium stearate.

The parenteral or oral pharmaceutical compositions described above are favorably prepared into unit formulations that comply with the dosage of the active element. Examples of such dosage unit formations include tablets, pills, capsules, injections (ampoules) and suppositories. Preferably, the content of the antibody is usually about 5-500 mg per dosage unit formulation, specifically about 5-100 mg for an injection and about 10-250 mg for other formulations. Each of the compositions mentioned above may contain other active element as long as no unfavorable interaction occurs with the antibody upon blending them.

Although a dosage of a pharmaceutical agent as a prophylactic/therapeutic agent comprising the antibody of the invention differs depending on the subject of administration, the target disease, the symptom, the administration route and the like, when it is used, for example, for treating adult type 2 diabetes, the antibody of the invention is preferably administered as an intravenous injection for usually about 0.01-20 mg/kg (weight), preferably about 0.1-10 mg/kg (weight), more preferably about 0.1-5 mg/kg (weight) at a time for about 1-5 times a day, preferably for about 1-3 times a day. Furthermore, when it is used for treating adult obesity, the antibody of the invention is preferably administered as an intravenous injection for usually about 0.01-20 mg/kg (weight), preferably about 0.1-10 mg/kg (weight), more preferably about 0.1-5 mg/kg (weight) at a time for about 1-5 times a day, preferably for about 1-3 times a day. These dosages may similarly apply to administration for the prevention of type 2 diabetes and obesity, or for other parenteral (e.g., subcutaneous) or oral administration. When the symptom is particularly severe, the dosage may be increased accordingly.

Herein, abbreviation of the amino acids or the like are those following IUPAC-IUB Commission on Biochemical Nomenclature or those common in the art, whose examples are shown below. If any enantiomer exists for an amino acid, it is shown in a L-form rather than in a R-form, unless otherwise noted.
Gly: glycine Ala: alanine
Val: valine Leu: leucine
Ile: isoleucine Ser: serine
Thr: threonine Cys: cysteine
Met: methionine Glu: glutamic acid
Asp: aspartic acid Lys: lysine
Arg: arginine His: histidine
Phe: phenylalanine Tyr: tyrosine
Trp: tryptophan Pro: proline
Asn: asparagine Gln: glutamine
PAM: phenylacetamidomethyl
Boc: t-butyloxycarbonyl
Fmoc:9-fluorenylmethyloxycarbonyl
Cl-Z: 2-chloro- benzyloxycarbonyl
Bge-Z: 2-bromo-benzyloxycarbonyl
Bzl: benzyl
Cl-Bzl: 2-chloro-benzyl
OcHex: cyclohexyl ester
OBzl: benzyl ester
Tos: p-toluenesulfonyl
HONB: N-hydroxy-5-norbornene-2,3-dicarboxyimide
HOBt: 1-hydroxybenzotriazole
HOOBt: 3-hydroxy-3,4-dihydro-4-oxo-1,2,3-benzotriazine
MeBzl: 4-methylbenzyl
Bom: benzyloxymethyl
Bum: t-butoxymethyl
Trt: trityl
DNP: dinitrophenyl
TFA: trifluoroacetic acid
DMF: N,N-dimethylformamide
DCM: dichloromethane
DCC: N,N'-dicyclohexylcarbodiimide
BHA: benzhydrylamine
pMBHA: p-methyl benzhydrylamine
CHO: formyl

Hereinafter, the present invention will be described more specifically by means of examples, although the present invention should not be limited thereto.

### [EXAMPLE 1]

### <Materials and Methods>

### 1. Chemotaxis assay

Mouse skeletal muscle-derived myoblast cell line C2C12 (obtained from the American Type Culture Collection (ATCC)) and human monocytic leukemia cell line THP-1 (obtained from the Health Science Research Resources Bank, HSRRB), each obtained from an official cell bank, were each cultured in a Dulbecco's modifies Eagle's medium (DMEM) containing 10% fetal bovine serum or an RPMI-1640 medium containing 10% fetal bovine serum, and the cells at the log growth phase were used for the experiments. 2 x 10⁵ C2C12 cells were seeded on a 100-mm plate, cultured at 37°C overnight, added with forskolin (Sigma) to a final concentration of 20 µM and cultured at 37°C for another two days. 10⁶ THP-1 cells were seeded on a 100-mm plate, simultaneously added with forskolin (Sigma) to a final concentration of 20 µM and cultured at 37°C for two days. The forskolin is a reagent that increases the intracellular cAMP concentration, which activates A kinase and the transcription factors downstream therefrom. A forskolin-stimulated THP-1 cell line is widely used for immunological research as a cell line that has a similar nature to that of an activated macrophage.

550 µl each of mouse CXCL14 (R&D Systems) and human CXCL14 (Peprotec) diluted with a chemotaxis buffer [0.1% Fatty acid-free BSA (Sigma)-20 mM HEPES pH 8.0 (Invitrogen)-DMEM] to a final concentration of 100 nM were added to 24-well plates containing the C2C12 cells and the THP-1 cells, respectively. For neutralization experiments with antibodies, sheep anti-mouse CXCL14 polyclonal antibody (AF730, R&D Systems) or rat anti-mouse CXCL14 monoclonal antibody (MAB730, R&D Systems) was added to the chemotaxis buffer containing CXCL14 to a final concentration of 10 µg/ml. Next, Chemotaxicell microchambers (Kurabo) with pore sizes of 8 µm and 5 µm for the C2C 12 cells and the THP-1 cells, respectively were left to stand still on the reaction solutions. The forskolin-stimulated C2C12 cells or the forskolin-stimulated THP-1 cells were washed once in a chemotaxis buffer to remove the fetal bovine serum. Thereafter, 2 x 10⁵ cells/200 µL were layered on the Chemotaxicell microchambers. The resulting 24-well plates were cultured at 37°C for 6 hours (C2C12 cells) or 2 hours (THP-1 cells). At the end of the reaction, the culture solutions as the upper layer in the microchambers were immediately removed and cells attached to the bottom of the microchamber were fixed and stained with Diff-Quik (International Reagents). Cells remaining at the upper layer of the microchambers were removed with a cotton bud. The membranes of the microchambers were removed and mounted on glass slides to count the number cells that migrated around the pores under an optical microscope (100x). The measurements were carried out with n = 4 or more subjects for each sample and StatView-J5.0 (SAS Institute) was run for statistical processing.

### 2. Western Blot Analysis

The C2C12 myoblast cell line was cultured in a DMEM medium containing 5% horse serum for four days to induce muscle differentiation. Following cultivation in a serum-free DMEM medium at 37°C for 16 hours, the myocytes were treated with 100 nM mouse CXCL14 (R&D Systems) for an hour with further addition of 10 nM insulin (Sigma) for stimulation at 37°C for 10 minutes. For neutralization experiments with antibodies, sheep anti-mouse CXCL14 polyclonal antibody (AF730, R&D System) was added to a pre-treated medium containing CXCL14 to a final concentration of 10 µg/ml. As a negative control, a medium containing purified IgG from a non-immunized sheep was used. These cells were dissolved, and 20 µg of the protein mixture was subjected to SDS-polyacrylamide gel electrophoresis, followed by western blot analysis with anti-Akt-pSer473 antibody and anti-Akt antibody (Cell signaling). Akt is serine/threonine kinase also known as protein kinase B (PKB) which is activated in PI3 kinase pathway and involved in various phenomena such as insulin metabolism.

### <Results and Discussion>

The chemotaxis, i.e., a typical biological activity, of CXCL14 was analyzed by an *in vitro* assay using Chemotaxicell microchamber. As has been reported previously (Nara N et al., J. Biol. Chem., vol. 282, pp. 30794-30803, 2007), muscle-differentiated C2C12 cells react with CXCL14. Hence, the cells were subjected to a chemotaxis assay following stimulation of the C2C12 cells with forskolin, a general differentiation inducing reagent. As a result, cell attraction by mouse CXCL14 was detected (Figure 1). A similar activity was also detected for human CXCL14 (Figure 1). Since only two residues among the 77 amino acids consisting their polypeptides were different between the mouse CXCL14 and human CXCL14 (see SEQ ID NOS: 1 and 2), the receptors expressed in the C2C12 cells were expected to have cross-reacted with the human CXCL14. Next, mouse CXCL14-specific antibodies were added to the chemotaxis assay solutions, where attraction of C2C12 cells by mouse CXCL14 was inhibited by 61% and 54% with a polyclonal antibody AF730 and a monoclonal antibody MAB730, respectively (Figure 2). Subsequently, the forskolin-stimulated THP-1 cells were used for the same chemotaxis assay, where cell attraction by CXCL14 was inhibited by 79% by addition of anti-mouse CXCL14 polyclonal antibody AF730 (Figure 3). These results prove that addition of anti-CXCL14-specific antibody can neutralize biological activity of CXCL14 on two types of cells as target cells of CXCL14 (skeletal myocytes and activated macrophages) involved in induction of obese insulin resistance. In particular, since activated macrophages that accumulate in visceral fat are known as a major aggravating factor of obese diabetes, the anti-CXCL14 antibody capable of suppressing the chemotaxis of the activated macrophages was found to be useful as an antidiabetic agent. When an anti-mouse CXCL14 monoclonal antibody MAB730 was added to an assay system of THP-1 cell, chemotaxis of THP-1 cells to human CXCL14, to the contrary, increased (Figure 4). The mechanism of this phenomenon is yet unknown but it indicates that the CXCL14-specific monoclonal antibody possibly controls the biological activity of CXCL14 in both negative and positive manners.

In a differentiated C2C12 myocytes, phosphorylation (activated state) of the serine residue at position 473 of the Akt kinase due to insulin stimulation is known to be partially inhibited by pre-treatment with CXCL14 (Nara N et al., J. Biol. Chem., vol. 282, pp. 30794-30803, 2007). Thus, we studied whether an anti-CXCL14 antibody can neutralize the insulin signal inhibitory activity by CXCL14. In the presence of a control antibody, the band of phosphorylated Akt weakened by pre-treatment with mouse CXCL14 whereas the band of phosphorylated Akt was still strong in the presence of anti-mouse CXCL14 polyclonal antibody AF730 (Figure 5). This result indicates that addition of anti-CXCL14-specific antibody can neutralize the insulin signal inhibitory activity by CXCL14 in the myocytes. Myocytes are major tissue that is responsible for the sugar level regulation by insulin. Since a CXCL14 expression level in the myocytes increases along with obesity, a partial inhibition of insulin signal by CXCL14 also seemed to contribute to systemic insulin resistance by obesity. The anti-CXCL14 antibody was expected to be effective to a secondary activity of CXCL 14 associated with onset of diabetes.

In studies using forskolin-stimulated C2C12 cells, cell attraction by mouse CXCL14 was pertussis toxin-sensitive which was similar to the response to a different type of chemokine CXCL12. Therefore, a CXCL14 receptor appears to be a trimeric G protein-coupled seven-transmembrane protein, although no CXCL14 receptor gene has been isolated or identified so far in any species including human and mouse. The anti-CXCL14-specific antibody is not only useful as a tool for alleviating pre-diabetic state caused by CXCL14 excessively produced by obesity but also expected to be useful in clarifying the yet unknown physiological functions and receptor structure of CXCL14.

### INDUSTRIAL APPLICABILITY

The present invention provides an antibody that recognizes a compound that suppresses or inhibits BRAK (CXCL14) functions, more particularly an antibody (preferably, a human monoclonal antibody) that specifically recognizes BRAK. The invention also provides a hybridoma cell that produces the antibody and a method for producing the antibody. In addition, the present invention provides, as use of the compound mentioned above (particularly, an anti-BRAK antibody), a diagnostic agent for type 2 diabetes and obesity, an agent for preventing and/or treating type 2 diabetes and obesity, an agent that decreases the number of macrophages in white adipose tissue, or an agent that ameliorates insulin resistance, a method for preventing and/or treating type 2 diabetes and obesity, a method for decreasing the number of macrophages in white adipose tissue and a method for ameliorating insulin resistance.

The compound mentioned above (particularly, an anti-BRAK antibody) is extremely useful in that it can be used as a novel therapeutic agent for type 2 diabetes as well as an agent for diagnosing the risk of type 2 diabetes and obesity, and further as a possible agent for relieving obesity.

## Claims

1. An antibody that recognizes a polypeptide comprising an amino acid sequence represented by SEQ ID NO: 1 or 2, a derivative thereof or a partial peptide thereof.

2. The antibody according to claim 1, which is a monoclonal antibody.

3. The antibody according to claim 1, which is a humanized antibody or a human antibody.

4. The antibody according to claim 1, which is labeled.

5. The antibody according to claim 1, which has an activity of decreasing the number of macrophages in white adipose tissue

6. The antibody according to claim 1, which has an activity of ameliorating insulin resistance.

7. A hybridoma cell that produces the antibody according to claim 1.

8. A method for producing the antibody according to claim 1, comprising: culturing the hybridoma cell according to claim 7 *in vivo* or *in vitro;* and collecting the antibody according to claim 1 from the body fluid or the culture.

9. A diagnostic agent comprising the antibody according to claim 1.

10. The diagnostic agent according to claim 9, which is used for diagnosing type 2 diabetes and/or obesity.

11. A pharmaceutical agent comprising the antibody according to claim 1.

12. The pharmaceutical agent according to claim 11, which is an agent for preventing and/or treating type 2 diabetes and/or obesity.

13. The pharmaceutical agent according to claim 11, which is an agent that decreases the number of macrophages in white adipose tissue.

14. The pharmaceutical agent according to claim 11, which is an agent that ameliorates insulin resistance.

15. A method for preventing and/or treating type 2 diabetes and/or obesity, comprising administering an effective amount of the antibody according to claim 1 to a mammal.

16. A method for decreasing the number of macrophages in white adipose tissue, comprising administering an effective amount of the antibody according to claim 1 to a mammal.

17. A method for ameliorating insulin resistance, comprising administering an effective amount of the antibody according to claim 1 to a mammal.

18. Use of the antibody according to claim 1 for producing an agent for diagnosing type 2 diabetes and/or obesity.

19. Use of the antibody according to claim 1 for producing an agent for preventing and/or treating type 2 diabetes and/or obesity.

20. Use of the antibody according to claim 1 for producing an agent for decreasing the number of macrophages in white adipose tissue.

21. Use of the antibody according to claim 1 for producing an agent for ameliorating insulin resistance.
